Europäisches Patentamt

European Patent Office

Office européen des brevets

(19)

(11) Publication number: **0 119 992**
A2

(12)

# EUROPEAN PATENT APPLICATION

(21) Application number: **84890056.9**

(22) Date of filing: **21.03.84**

(51) Int. Cl.³: **A 23 K 1/18**, A 23 K 1/175

(30) Priority: **22.03.83 US 477743**

(43) Date of publication of application: **26.09.84**
**Bulletin 84/39**

(84) Designated Contracting States: **AT BE CH DE FR GB IT LI LU NL SE**

(71) Applicant: **UNION CARBIDE CORPORATION, Old Ridgebury Road, Danbury Connecticut 06817 (US)**

(72) Inventor: **Frost, Albert Carl, 73 New York Avenue, Congers New-York 10920 (US)**
Inventor: **Sherman, John Delano, 91 Valley View Road, Chappaqua New-York 10514 (US)**
Inventor: **Manchanda, Krishan Dayal, 63 Judith Drive, Danbury Connecticut 06810 (US)**

(74) Representative: **Berger, Erhard, Dr., Slebensterngasse 39 Postfach 306, A-1071 Wien (AT)**

(54) **Feeding poultry.**

(57) Poultry feeds containing a chabazite-containing material provide improved weight gain and feed efficiency.

EP 0 119 992 A2

## FEEDING POULTRY

### FIELD OF THE INVENTION:

The instant invention relates to the feeaing poultry by the use of a chabazite containing feed.

### BACKGROUND OF THE INVENTION:

The use of zeolitic materials in poultry feeds has been of limited interest in recent years with most attention being directed to the zeolite clinoptilolite.

"Paper No. 10, Utilization of Zeolites in Agriculture", (Background Papers for Innovative Biological Technologies for Less Developed Countries, Office of Technology Assessment Workshop, November 24, 25, 1980, Report for the Committee on Foreign Affairs, U.S. House of Representatives, September 1981, U.S. Governmental Printing Office, Washington D.C., 1981) is representative of the work done in the development of feeding chickens. The paper discloses the use of clinoptilolite ana mordenite in a feed for Leghorn chickens. The zeolite-containing feed provided a higher feea etficiency than the reea which aid not contain the zeolite.

The use of various mineral additives to provide minerals and/or grit to the diet of poultry has been aisclosed. U.S. Patent No. 1,399,206 discloses the use of slag from iron furnaces. Furnace slag comprises a wide range of components and is provided as a form of grit for the gizzara of poultry. U.S. Patent No. 1,867,063 discloses the use of bentonite in a wet poultry or stock feed.

D-13,862

The bentonite is provided to the feed to aid in suspending feed particles which may otherwise precipitate. U.S. Patent No. 2,162,609 discloses the use of hydro-silicates of aluminum, e.g. bentonite, in a poultry feed. The bentonite is provided as a gell-forming substituent. U.S. Patent No. 2,486,426 discloses the use of a light-reflecting substance in a feed for fowl. The light-reflecting substance is an expanded vermiculite and is added to induce fowl to eat the light-reflecting material and concurrently to ingest food. U.S. Patent No. 3,152,573 discloses the use of two sizes of glass beads in a poultry feed. This combination of beads is suggested to aid in the digestion of food by fowl. U.S. Patent No. 3,687,680 discloses the use of montmorillonite clay in a poultry feed. The montmorillonite clay is disclosed as advantageous owing to the fact that it is low in sodium, high in calcium, low in silica and low in aluminum. A typical composition of montmorillonite is disclosed at column 3, lines 10 to 44 of the patent. U.S. Patent No. 3,776,188 discloses the use of dried ferrous sulfate hepta-hydrate for control of the stench which results from the droppings of fowl. The ferrous sulfate hepta-hydrate is generally used with fly ash or dried fine zeolite powders, e.g., as an adsorbent. U.S. Patent No. 3,812,269 discloses the use of propionic acid and one of activated synthetically formed silicic acid or silicate as a feed preservative. U.S. Patent No. 3,807,981 discloses the use a polymetallic phosphate glass containing Fe, Zn, Mn ($MnCO_3$), CuO, $CoCl_2$, $Na_2HPO_4$ and $NaH_2PO_4$ as an animal feed

D-13,862

additive. U.S. Patent No. 3,836,676, similar to
U.S. Patent No. 3,776,188, discloses the use of
ferrous sulfate hepta-hydrate for the control of the
odor of poultry droppings. The ferrous sulfate
hepta-hydrate may be used with fly ash or powdered
zeolite.

The above patents are representative of the
studies which have been carried out on the use of
zeolites and other additives in poultry feeds.
These studies are few and far between and tend to
show that no direction can be gleened from tne
studies performed. Further, the studies have
grouped together all poultry as being the same. Tne
inventors herein have found that this arbitrary
grouping is not necessarily justified.

SUMMARY OF THE INVENTION:

The instant invention relates to a process
for raising poultry, preferably turkeys, witn
improved weight gain ana teed efficiency using a
feed that contains chabazite or a chabazite-
containing material as compared over the same
growing period with a non-chabazite-containing feed.

DETAILED DESCRIPTION OF THE INVENTION:

The instant invention relates to a process
for raising poultry, preferably turkeys, by the
addition of a chabazite-containing material to the
poultry feed. The term "chabazite-containing
material" is used herein to refer to any material
which contains chabazite. Chabazite is a naturally
occurring zeolite. Tne physical and cnemical
characteristics of chabazite are disclosed on page
138 of Zeolite Molecular Sieves, by Donald W. Breck, John Wiley and Sons, New York, 1974, said page being incorporated herein by reference thereto.

The raising of poultry is well documented in the art. For example, the raising of turkeys is generally related to the various growth stages and is generally carried out by providing pre-starter turkeys (up to 4 weeks old) with a feed material having the predetermined level of protein and other constituents. The feed is typically adjusted as the turkeys age to provide lower protein levels.

The feed material, prior to addition of the chabazite-containing material, can comprise any of the routinely employed feed materials which have sources of carbohydrates, protein, fat, fiber, minerals, etc. The feeds for poultry generally include at least one of wheat, barley, soybean, corn meat and/or bone materials, flax, fishmeal, peanut meal, sugar beet pulp, cotton seed hull and meal, sugar cane pulp (bagasse), sorghum, milo, rye, rice and the like and mixtures thereof. The exact nature of the feed is not critical. The feeds generally employed in the raising of poultry have a minimum crude protein of at least 10.0 percent, a minimum crude fat of 4.0 percent and a minimum crude fiber analysis of 2.0 percent, with all percentages being on a weight basis based on the total feed weight. The feed preferably contains at least about 16% by weight protein and at least 5.0% by weight fat. Additionally, the feed typically contains vitamins, disease inhibitors (typically antibiotics) and various minerals. The various analysis (protein, carbohydrate-fat and fiber) and additives employed in poultry feeds are well known to the art as shown by reference to U.S. Patent No. 4,178,368, column 5,

D-13,862

line 56 to column 6, line 21. The actual nature of the feed is not narrowly critical to the instant invention with a wide range of feeds being employable herein.

The chabazite-containing material most likely to be employed in feeding poultry in accordance with the instant invention will be a naturally occurring chabazite ore, although chabazite ores subjected to secondary purification(s) and treatment(s) also come within the meaning of the term "chabazite-containing material". When the chabazite-containing material is a naturally occurring chabazite ore it will contain various components other than chabazite. The chabazite-containing material can be further characterized as to the relative amount of chabazite present in the chabzite-containing material in relation to other zeolitic components by a chabazite weight ratio to other zeolitic components of at least 1.5 to one and is preferably 2 to 1 or greater. Further, it is believed that mixtures of chabazite and erionite and/or clinotilolite may synergistically interact to provide mixtures which provide improved performance as poultry feed additives which is greater than the individual zeolitic components, although the exact nature of such synergistic interaction is not understood at present.

The chabazite-containing material can be most any material that contains chabazite as a chabazite component part which when employed in an effective amount in feeding poultry provides for an improved weight gain and feed efficiency by the poultry as compared with a similar feed which does not contain the chabazite-containing material. In addition, the chabazite-containing material can be subjected to one or more secondary treatment processes, if desired, prior to use in the process of the instant invention. A representative chemical analysis of an untreated chabazite-containing ore is as follows:

| Chemical Analysis* | Wt. % (Range) |
|---|---|
| $SiO_2$ | 50 - 70 |
| $Al_2O_3$ | 10 - 30 |
| CaO | 1 - 10 |
| MgO | 0.8 - 3.0 |
| $Na_2O$ | 0.9 - 9.1 |
| $K_2O$ | 0.4 - 3.5 |
| $Fe_2O_3$ | 2.7 - 6.0 |

* moisture-free basis

The following examples are provided to illustrate the invention and are not intended to be limiting thereof.

### EXPERIMENTAL RESULTS

Chabazite-containing turkey feeds were evaluated to demonstrate their utility in improving the weight gain and feed efficiency of turkeys by carrying out a feeding study on 480 male (tom) Longmont turkeys.

The turkeys were randomly divided into twenty four (24) subgroups of twenty each. The twenty four subgroups (numbered 1 through 24) were then randomly divided into four larger groups, i.e. each new group comprised six (6) of the first twenty four (24) subgroups. These groups (containing 120 turkeys) were then designated as Groups A, B, C and

D-13,862

0119992

D. Group A was designated the control group and was not fed a feed containing a zeolitic material. Groups B, C and D were evaluated for their affect on weight gain and feed efficiency wherein the feed fed Groups B, C and D after the eight week contained zeolites Na-A (sodium exchanged zeolite A), Ca-A (calcium exchanged zeolite A) and a chabazite ore,* respectively, in an amount of 2 percent by weight, based on the total weight of the feed (the zeolite component having replaced an equivalent amount of corn).

The chabazite-containing ore had the following chemical analysis (moisture-free): $SiO_2$ (63.8); $Al_2O_3$ (15.8); $CaO$ (5.6; $MgO$ (1.48); $Na_2O$ (2.2); $K_2O$ (1.4); $Fe_2O_3$ (4.3) and had an oxygen adsorption of 87.1% of that obtained for pure chabazite at a P/Po of 0.48 at 77°K.

Groups A, B, C and D were fed five different feeds (designated Feeds 1, 2, 3, 4 and 5) based upon the age of the turkeys being fed. The turkeys were allowed to consume as much feed as desired and the amount consumed was measured. The analyses of the feeds and the corresponding application period to the turkeys being fed are set forth in Table I. The turkeys in the four groups were weighed after 8, 12 and 20 weeks and the weight gain and the feed efficiency (for the periods from the end of week 8 to the end of week 12 and from the end of week 8 to the end of week 20) calculated. The weight gain for a given group of poultry for a given period was calculated as the difference between the weight of the poultry in the group at the end of a feeding period and the weight of the poultry at the beginning of the next referenced

D-13,862 * Ground to an effective size of 100 wt. % less than 100 U.S. mesh, 95 wt. % less than 200 U.S. mesh and 55 wt. percent less than 325 U.S. mesh.

feeding period. The feed efficiency for a given group of poultry was calculated as the pounds of feed required to achieve a one pound weight gain per group member. The weights of the poultry in Groups A, B, C & D were measured at the end of the eighth week. Groups B, C and D were then put on the zeolite diets, as above described. Table II shows that there was a weight gain enhancement during the period between the end of week 8 and week 12 for the turkeys fed the chabazite-containing feed (Group D) as compared with turkeys fed the non-chabazite-containing feed (Group A). Table III shows that for the period between the end of week 8 and the end of week 20 that the turkeys in Group D showed improved weight gain and improved feed efficiency as compared with the turkeys in Groups A, B or C. Thus, the chabazite-containing feed resulted in improved weight gain for turkeys during the growth period after the eighth week of the turkey growth period, i.e. after the turkeys were eight weeks old.

The mortality rate and litter moisture of turkeys' droppings were evaluated to determine the effect, if any, of chabazite-containing materials on such. Tables IV and V show that a feed containing a chabazite-containing material has little effect on mortality and litter moisture of droppings, i.e. the mortality is not statistically affected and the droppings have essentially no difference in moisture content.

Although additional studies have not been completed to date, it is believed the weight gain observed by the use of a turkey feed containing a chabazite-containing feed may be greater for tom

D-13,862

(male) turkeys as compared to female turkeys, although the reason for this difference is not clearly understood. Further, feed studies have been carried out on turkeys and chickens but due to the statistical error limits of these studies the results are not statistically meaningful, although the same improved weight gains are expected to occur with other species of poultry when fed chabazite-containing poultry feeds.

TABLE I[1]

| Feed Identity:<br>Weeks of Age: | FEED 1<br>(up thru 3) | FEED 2<br>(4 thru 8) | FEED 3<br>(9 thru 12) | FEED 4<br>(13 thru 16) | FEED 5<br>(16 thru 20)[3] |
|---|---|---|---|---|---|
| Percent Protein: | 28 | 25 | 22 | 19 | 16 |
| Crude Fiber (%) | 3.78 | 3.50 | 3.23 | 2.83 | 2.45 |
| Fat (%) | 7.0 | 6.56 | 6.73 | 6.0 | 6.40 |
| Kilocalories/pound[4]: | 1332 | 1359 | 1390 | 1442 | 1481 |
| Ingredients[2] | | | | | |
| Soybean: | 960.0 | 815.0 | 700.0 | 470.0 | 290.0 |
| Ground Corn: | 776.4 | 942.9 | 1060.0 | 1305.0 | 1500.0 |
| Meat & Bone Scrap: | 120.0 | 120.0 | 120.0 | 120.0 | 120.0 |
| Fat, Soybean Oil: | 90.0 | 75.0 | 75.0 | 65.0 | 55.0 |
| Calcium diphosphate: | 34.0 | 24.3 | 23.0 | 22.3 | 18.0 |
| Vitamins: | 10.0 | 10.0 | 10.0 | 10.0 | 10.0 |
| Salt | 5.0 | 5.0 | 5.0 | 5.0 | 6.0 |
| Preservative | 3.7 | 1.9 | .8 | 1.1 | .7 |
| Trace Minerals | 1.0 | 1.0 | 1.0 | 1.0 | 1.0 |
| Limestone | -- | 4.9 | 5.0 | -- | -- |
| TOTAL FEED WEIGHT | 2000.10 | 2000.00 | 1999.80 | 1999.40 | 2000.70 |

[1]   When zeolite was added to each feed an equivalent amount of ground corn was removed to provide the total feed weight.

[2]   Given in pounds per ton (English) of feed.

[3]   Turkeys were slaughtered at the end of week 20.

[4]   Metabolizable energy.

| TABLE II | Subgroup | No. | No. | Mortality | | 8 Week | 12 Week | |
|---|---|---|---|---|---|---|---|---|
| Group A | No. | Started | Weighed | No. | % | Wt. | Wt. | Gain [1,2,3] |
| | 2 | 20 | 20 | 0 | 0 | 2945 | 6033 | 3088 |
| | 5 | 20 | 20 | 0 | 0 | 2910 | 5988 | 3078 |
| | 10 | 20 | 20 | 0 | 0 | 2875 | 5974 | 3099 |
| | 13 | 20 | 20 | 0 | 0 | 3005 | 6001 | 2996 |
| | 17 | 20 | 20 | 0 | 0 | 2805 | 5897 | 3092 |
| | 22 | 20 | 20 | 0 | 0 | 2905 | 6033 | 3128 |
| Total & Ave. | | 120 | 120 | 0 | 0 | 2908 | 5988 | 3080 |
| Group B | 1 | 20 | 19 | 1 | 5.0 | 3025 | 6059 | 3034 |
| | 7 | 20 | 20 | 0 | 0 | 2890 | 5874 | 2984 |
| | 9 | 20 | 20 | 0 | 0 | 3010 | 6056 | 3046 |
| | 16 | 20 | 20 | 0 | 0 | 3030 | 6056 | 3026 |
| | 19 | 20 | 18 | 2 | 10.0 | 2820 | 6048 | 3228 |
| | 21 | 20 | 19 | 1 | 5.0 | 3045 | 6159 | 3114 |
| Total & Ave. | | 120 | 116 | 4 | 3.3 | 2970 | 6042 | 3072 |
| Group C | 4 | 20 | 20 | 0 | 0 | 2910 | 5965 | 3055 |
| | 8 | 20 | 20 | 0 | 0 | 2890 | 5919 | 3026 |
| | 12 | 20 | 20 | 0 | 0 | 2945 | 6001 | 3056 |
| | 14 | 20 | 19 | 1 | 5.0 | 2885 | 6016 | 3131 |
| | 18 | 20 | 19 | 1 | 5.0 | 2790 | 5879 | 3089 |
| | 24 | 20 | 18 | 2 | 10.0 | 2770 | 5897 | 3127 |
| Total & Ave. | | 120 | 116 | 4 | 3.3 | 2865 | 5946 | 3081 |
| Group D | 3 | 20 | 19 | 1 | 5.0 | 3080 | 6207 | 3127 |
| | 6 | 20 | 19 | 1 | 5.0 | 2890 | 6116 | 3226 |
| | 11 | 20 | 20 | 0 | 0 | 3065 | 6237 | 3172 |
| | 15 | 20 | 19 | 1 | 5.0 | 3055 | 6181 | 3126 |
| | 20 | 20 | 19 | 1 | 5.0 | 2935 | 6090 | 3155 |
| | 23 | 20 | 20 | 0 | 0 | 2880 | 6147 | 3267 |
| Total & Ave. | | 120 | 116 | 4 | 3.3 | 2984 | 6163 | 3179 |

[1] Gain for 4 week period between weeks 8 and 12 expressed in pounds.
[2] No improvement in feed efficiency observed based on error limits of the study based on a 95% confidence limit.
[3] Statistically significant difference (P ≤ 0.05)

## TABLE III

| Group A | Subgroup No. | 8 Week Weights | 20 Week Weights | Gain 8-20[1] Weeks | Feed/Bird 8-20 Weeks | Feed Efficiency[1] |
|---|---|---|---|---|---|---|
| | 2 | 2945 | 12800 | 9855 | 21.78 | 2.210 |
| | 5 | 2910 | 12320 | 9410 | 20.80 | 2.210 |
| Control | 10 | 2875 | 12310 | 9435 | 20.76 | 2.200 |
| | 13 | 3005 | 12650 | 9645 | 21.51 | 2.230 |
| | 17 | 2805 | 12400 | 9595 | 21.01 | 2.190 |
| | 22 | 2905 | 12800 | 9895 | 21.77 | 2.200 |
| | Average | 2908 | 12547 | 9639 | 21.30 | 2.210 |
| Group B | 1 | 3025 | 11400 | 8375 | 22.36 | 2.670 |
| | 7 | 2890 | 10600 | 7710 | 20.35 | 2.640 |
| | 9 | 3010 | 11470 | 8460 | 20.13 | 2.380 |
| Zeolite NaA | 16 | 3030 | 10860 | 7830 | 20.44 | 2.611 |
| | 19 | 2820 | 11000 | 8180 | 21.43 | 2.620 |
| | 21 | 3045 | 11450 | 8405 | 22.44 | 2.670 |
| | Average | 2970 | 11130 | 8160 | 21.20 | 2.599 |
| Group C | 4 | 2910 | 10400 | 7490 | 18.95 | 2.530 |
| | 8 | 2890 | 10510 | 7620 | 22.48 | 2.950 |
| Zeolite CaA | 12 | 2945 | 10760 | 7815 | 21.41 | 2.740 |
| | 14 | 2885 | 11121 | 8236 | 20.84 | 2.530 |
| | 18 | 2790 | 10710 | 7920 | 19.01 | 2.400 |
| | 24 | 2770 | 11180 | 8410 | 22.62 | 2.690 |
| | Average | 2865 | 10780 | 7915 | 20.90 | 2.640 |
| Group D | 3 | 3080 | 13400 | 10320 | 22.70 | 2.200 |
| | 6 | 2890 | 13181 | 10291 | 22.64 | 2.200 |
| | 11 | 3065 | 13340 | 10275 | 22.61 | 2.200 |
| | 15 | 3055 | 13290 | 10235 | 22.21 | 2.170 |
| | 20 | 2935 | 12960 | 10025 | 21.75 | 2.170 |
| | 23 | 2880 | 13100 | 10220 | 22.38 | 2.190 |
| | Average | 2984 | 13211 | 10227 | 22.38 | 2.188 |

[1]Statistically significant difference (P ≤ 0.05).

## TABLE IV

| Group | Subgroup | No. Weighed | Mortality No. | Mortality Percent |
|-------|----------|-------------|---------------|-------------------|
| A | 2 | 20 | 0 | 0 |
| | 5 | 20 | 0 | 0 |
| | 10 | 19 | 1 | 5.0 |
| | 13 | 18 | 2 | 10.0 |
| | 17 | 19 | 1 | 5.0 |
| | 22 | 19 | 1 | 5.0 |
| | | 115 | 5 | 4.2% |
| B | 1 | 19 | 1 | 5.0 |
| | 7 | 18 | 2 | 10.0 |
| | 9 | 17 | 3 | 15.0 |
| | 16 | 20 | 0 | 0 |
| | 19 | 19 | 1 | 5.0 |
| | 21 | 19 | 1 | 5.0 |
| | | 112 | 8 | 6.7% |
| C | 4 | 17 | 3 | 15.0 |
| | 8 | 18 | 2 | 10.0 |
| | 12 | 18 | 2 | 10.0 |
| | 14 | 18 | 2 | 10.0 |
| | 18 | 19 | 1 | 5.0 |
| | 24 | 16 | 4 | 20.0 |
| | | 106 | 14 | 11.7% |
| D | 3 | 18 | 2 | 10.0 |
| | 6 | 18 | 2 | 10.0 |
| | 11 | 19 | 1 | 5.0 |
| | 15 | 19 | 1 | 5.0 |
| | 20 | 19 | 1 | 5.0 |
| | 23 | 19 | 1 | 5.0 |
| | | 112 | 8 | 6.7% |

## TABLE V

| Week No. | Group* | | | |
|---|---|---|---|---|
| | A | B | C | D |
| 8 | 1 | 1 | 1 | 1 |
| 9 | 1 | 2 | 1 | 1 |
| 10 | 1 | 2 | 2 | 1 |
| 11 | 1 | 2 | 2 | 1 |
| 12 | 1 | 3 | 3 | 2 |
| 13 | 1 | 3 | 3 | 2 |
| 14 | 1 | 3 | 3 | 2 |
| 15 | 1 | 3 | 4 | 2 |
| 16 | 2 | 4 | 4 | 2 |
| 17 | 2 | 4 | 4 | 2+ |
| 18 | 2 | 4 | 4 | 2+ |
| 19 | 2 | 4 | 4 | 2+ |
| 20 | 2 | 4 | 4 | 2+ |

* The numerical values have the following
  qualitative meaning:
  1 = Dry - Normal
  2 = Slight Wet (Normal)
  3 = Wet
  4 = Very Wet

D-13,862

WHAT IS CLAIMED IS:

1.    The process of improving weight gain in the raising of poultry which comprises feeding poultry a feed containing an effective amount of a chabazite-containing material whereby the weight gain of the poultry is improved over that weight gain obtained by use of the feed without said chabazite-containing material when compared over the same period.

2.    The process of claim 1 wherein the chabazite-containing material is a chabazite ore and comprises the following chemical composition:

| Chemical Analysis* | Wt. % (Range) |
|---|---|
| $SiO_2$ | 50 - 70 |
| $Al_2O_3$ | 10 - 30 |
| $CaO$ | 1 - 6.2 |
| $MgO$ | 0.8 - 3.0 |
| $Na_2O$ | 0.9 - 9.1 |
| $K_2O$ | 0.4 - 3.5 |
| $Fe_2O_3$ | 2.7 - 6.0 |

3.    The process of claim 1 wherein the poultry are turkeys.

4.    The process of claim 3 wherein the turkeys are tom turkeys.

5.    The process of claim 3 wherein the feed efficiency is improved.

6.    The process of claim 3 wherein the period is the period after the eighth week of the turkey growth period.

7.    The process of claim 1 wherein the chabazite-containing material comprises a synergistic combination of chabazite and at least one of erionite and clinolitolite.

8. The process of claim 7 wherein the chabazito-containing material comprises a synergistic combination of chabazite and erionite.

9. The process of claim 8 wherein the weight ratio of chabazite to erionite is greater than 1.5 to 1.

10. The process of claim 1 wherein the chabazite-containing material consists essentially of chabazite.

11. A poultry feed for raising poultry comprising at least 10.0 percent by weight protein, at least 4.0 percent by weight fat, 2.0 percent by weight fiber and an effective amount of a chabazite-containing material.

12. The poultry feed of claim 11 wherein the chabazite-containing material consists essentially of a synergistic mixture of chabazite and erionite.

13. The poultry feed of claim 12 wherein the Chabazite-containing material consists essentially of a chabazite containing material.

14. The poultry feed of claim 11 wherein the feed contains at least .25% by weight chabazite.

15. The poultry feed of claim 14 wherein the feed contains at least 1.0% by weight chabazite.